(19) Europäisches Patentamt
European Patent Office
Office européen des brevets

(11) EP 1 561 451 A1

(12) DEMANDE DE BREVET EUROPEEN

(43) Date de publication:
**10.08.2005 Bulletin 2005/32**

(51) Int Cl.[7]: **A61K 7/032**

(21) Numéro de dépôt: **05290182.4**

(22) Date de dépôt: **27.01.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **03.02.2004 FR 0450198**

(71) Demandeur: **L'OREAL**
**75008 Paris (FR)**

(72) Inventeurs:
- **Bodelin, Sophie**
**92170 Vanves (FR)**
- **Lepeltier, Delphine**
**92120 Montrouge (FR)**

(74) Mandataire: **Boulard, Denis**
**L'OREAL - D.I.P.I.**
**25-29 Quai Aulagnier**
**92600 Asnières (FR)**

# (54) Composition cosmétique de maquillage pour les fibres kératiniques chargeante

(57) L'invention a pour objet un composition de revêtement des fibres kératiniques comprenant un milieu cosmétiquement acceptable caractérisée en ce qu'elle associe au moins un tensioactif ionique et au moins un tensioactif non ionique de HLB supérieur ou égal à 8 à 25 °C et en ce qu'elle présente :

- une teneur en matière sèche supérieure ou égale à 40% en poids et
- un profil adhésif inférieur ou égal à 3, ledit profil adhésif représentant le rapport du pouvoir adhésif au moment où 20% de ladite composition est évaporé ($PA_{20\%}$) sur le pouvoir adhésif à To (noté $PA_{T0}$, $T_0$ correspondant au moment de l'application de la composition).

L'invention a également pour objet l'utilisation d'une telle composition pour obtenir un maquillage chargeant des fibres kératiniques.

EP 1 561 451 A1

## Description

La présente invention a pour objet une composition cosmétique de revêtement des fibres kératiniques.

La composition selon l'invention peut être une composition de maquillage, encore appelée mascara, une base de maquillage des fibres kératiniques ou base-coat, une composition à appliquer sur un maquillage, dite encore top-coat, ou bien encore une composition de traitement des fibres kératiniques.
De préférence, la composition selon l'invention est une composition non rincée.

Plus spécialement, la composition selon l'invention est un mascara.
Par mascara, on entend une composition destinée à être appliquée sur les cils : il peut s'agir d'une composition de maquillage des cils, une base de maquillage des cils, une composition à appliquer sur un mascara, dite encore top-coat, ou bien encore une composition de traitement cosmétique des cils. Le mascara est plus particulièrement destiné aux cils d'êtres humains, mais également aux faux-cils.

Les compositions de maquillage pour les yeux, encore appelées « mascara » pour les cils sont généralement constituées d'une cire ou mélange de cires dispersée(s) à l'aide d'au moins un tensioactif dans une phase aqueuse contenant, par ailleurs, des polymères et des pigments.
C'est généralement à travers le choix qualitatif et quantitatif des cires et polymères que sont ajustées les spécificités d'application recherchées pour les compositions de maquillage, comme par exemple leur fluidité, leur pouvoir couvrant et/ou leur pouvoir recourbant. Ainsi, il est possible de réaliser diverses compositions qui, appliquées notamment sur les cils, induisent des effets variés de type allongement, recourbement et/ou épaississement (effet chargeant).
La présente invention vise plus particulièrement à proposer une composition utile pour réaliser un maquillage épais des fibres kératiniques et notamment des cils, dit encore maquillage chargeant. Au sens de la présente invention, le terme fibres kératiniques couvre les cheveux, les cils et les sourcils et s'étend également aux postiches et faux-cils synthétiques.
Avec les compositions de maquillage actuellement disponibles, cet effet est généralement obtenu par superposition de plusieurs couches de la composition de maquillage au niveau des fibres kératiniques et plus particulièrement des cils. Par ailleurs, dans le cas particulier des cils, cet effet est très souvent associé à une agglomération de plusieurs cils entre eux, c'est-à-dire à une non individualisation des cils.
Pour des raisons évidentes, il serait avantageux d'obtenir cet effet épaississant en une unique application et tout en obtenant une bonne séparation des cils.
Pour ce faire, il serait particulièrement intéressant de disposer d'une composition de maquillage qui soit suffisamment concentrée en matière sèche pour charger significativement le cil dès sa première mise en contact avec ladite composition et qui permette par ailleurs de gainer chaque cil de la teneur en matière sèche, on se heurte rapidement à un problème de défaut de fluidité. La composition de maquillage devient trop épaisse à l'application et ne présente plus la déformabilité nécessaire à son application homogène sur toute la surface des cils.

De manière inattendue, il a ainsi été constaté qu'il était possible de préparer des compositions présentant un extrait sec supérieur ou égale à 40% en poids grâce à la mise en oeuvre, dans ces compositions, d'un système d'émulsionnant spécifique associant un tensioactif ionique et un tensioactif non ionique de HLB supérieur ou égal à 8 à 25 °C, lesdites compositions présentant en particulier un pouvoir adhésif qui permet une bonne accroche sur les fibres kératiniques au moment de l'application sans rendre le dépôt trop collant au cours du séchage afin de conserver une application facile.

L'invention a ainsi pour objet, selon l'un de ses aspects, une composition de revêtement des fibres kératiniques comprenant un milieu cosmétiquement acceptable caractérisée en ce qu'elle associe au moins un tensioactif ionique et au moins un tensioactif non ionique de HLB supérieur ou égal à 8 à 25 °C et en ce qu'elle présente :

- une teneur en matière sèche supérieure ou égale à 40% en poids et
- un profil adhésif inférieur ou égal à 3, ledit profil adhésif représentant le rapport du pouvoir adhésif au moment où 20% de ladite composition est évaporé ($PA_{20\%}$) sur le pouvoir adhésif à To (noté $PA_{T0}$, $T_0$ correspondant au moment de l'application de la composition).

La présente invention vise également un procédé de maquillage des fibres kératiniques, caractérisé par le fait que l'on applique sur lesdites fibres une composition conforme à l'invention.

Elle se rapporte en outre à l'utilisation d'une composition conforme à l'invention pour obtenir un maquillage chargeant des fibres kératiniques et notamment des cils et des sourcils.

La présente invention a aussi pour objet l'utilisation de l'association d'au moins un tensioactif ionique et au moins un tensioactif non ionique de HLB supérieur ou égal à 8 à 25 °C pour obtenir une composition de revêtement des fibres kératiniques ayant une teneur en matière sèche supérieure ou égale à 40% en poids et un profil adhésif inférieur ou égal à 3, ladite composition permettant un maquillage chargeant des fibres kératiniques et notamment des cils et des sourcils.

Au sens de la présente invention, on entend qualifier par le terme « chargeant » la notion d'un maquillage épais et volumateur des fibres kératiniques, en particulier des cils.

La composition de revêtement des fibres kératiniques selon l'invention est avantageusement non solide. Par composition solide, on entend une composition qui ne s'écoule pas quelle que soit la contrainte mécanique appliquée, à température ambiante. Ce type de composition solide est par exemple un mascara solide, plus couramment appelé mascara pain. Pour maquiller les cils à partir d'un mascara pain, ce dernier doit être d'abord délité avec une brosse à mascara imprégnée d'eau et le mélange de mascara et d'eau ainsi préparé est ensuite appliqué avec la brosse sur les cils.

CARACTERISATION DE LA TENEUR EN MATIERE SECHE

La teneur en matière sèche, c'est à dire la teneur en matière non volatile, peut être mesurée de différentes manières, on peut citer par exemple les méthodes par séchage à l'étuve, les méthodes par séchage par exposition à un rayonnement infrarouge ainsi que les méthodes chimiques par titrage de l'eau selon Karl Fischer.
De préférence, la quantité de matière sèche, communément appelée « extrait sec » des compositions selon l'invention, est mesurée par échauffement de l'échantillon par des rayons infrarouges de 2 µm à 3,5 µm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler.
Cette technique est parfaitement décrite dans la documentation de l'appareil fournie par Mettler.
Le protocole de mesure est le suivant :
On étale environ 1g de la composition sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.
La teneur en matière sèche est calculée de la manière suivante :

Extrait Sec = 100 x (masse sèche / masse humide).

Les compositions selon l'invention se caractérisent par une teneur en matière sèche supérieure ou égale à 40 % en poids, notamment supérieure ou égale à 41 % en poids, de préférence encore supérieure ou égale à 42% en poids, mieux supérieur ou égal à 45% par rapport au poids total de la composition, pouvant aller jusqu'à 50%, voire 55% en poids.

Profil adhésif de la composition

Le profil adhésif de la composition est mesurée à température ambiante conformément au protocole suivant, à l'aide de l'analyseur de texture sous la dénomination TA-TX2i par la société RHEO sur un échantillon de la composition étalée sur une plaque de verre sous forme d'un film d'une épaisseur de 300µm.
L'essai consiste à mettre en contact une sonde cylindrique en élastomère de 6mm de diamètre avec le produit.
La sonde est appliquée (vitesse 0.1 mm/s) avec une force de 1 N sur le produit (pendant 10s) puis elle est retirée (vitesse = 1 mm/s). La force nécessaire pour retirer la sonde est mesurée. Le collant (pouvoir adhésif) correspond à l'intégrale de la courbe de la force en fonction du temps pour la partie de la courbe correspondant aux valeurs négatives de la force (force d'étirement).
Le pouvoir adhésif est exprimé en N.s.
La mesure est réalisée à To ($PA_{T0}$) et à 20% d'évaporation ($PA_{20\%}$) .Le profil adhésif est égal à $PA_{20\%}/PA_{T0}$. $PA_{T0}$ est la mesure du pouvoir adhésif à T0, après que la formule a été étalée sur la plaque préalablement tarée et la masse déposée mesurée avec une balance de précision AX204 de la société METTLER-TOLEDO. $PA_{20\%}$ est la mesure du pouvoir adhésif, après que 20 % de la composition appliquée sur la plaque de verre se soit évaporé, c'est-à-dire lorsque le film étalé sur la plaque de verre a perdu 20% de sa masse initiale : la masse du film, déterminée par le calcul, correspond alors à 80% de sa masse initiale . La mesure de $PA_{20\%}$ est faite dès que la balance affiche cette valeur.

La composition selon l'invention présente avantageusement un profil adhésif allant de 0,1 à 3, de préférence de 0,5 à 2,7, mieux de 1 à 2,5, encore mieux de 1,2 à 2,3 et notamment de 1,5 à 2.

Système émulsionnant

Selon l'invention, on utilise un système émulsionnant choisi de manière appropriée pour l'obtention d'une

émulsion huile-dans-eau ou cire-dans-eau. En particulier, on utilise un émulsionnant possédant à 25 °C une balance HLB (hydrophile-lipophile balance) au sens de GRIFFIN, supérieure ou égale à 8.
La valeur HLB selon GRIFFIN est définie dans J. Soc. Cosm. Chem. 1954 (volume 5), pages 249-256.
Les compositions selon l'invention peuvent contenir des agents tensioactifs émulsionnants présents notamment en une proportion allant de 0,1 à 40 %, et mieux de 0,3 % à 20 % en poids par rapport au poids total de la composition.
Ces agents tensioactifs peuvent être choisis parmi des agents tensioactifs non ioniques, anioniques, cationiques, amphotériques ou encore des émulsionnants tensioactifs. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.
Dans le cadre de la présente invention, ce système émulsionnant comprend au moins un tensioactif ionique et au moins un tensioactif non ionique de HLB supérieur ou égal à 8 à 25°C.

<u>Tensioactif non ionique de HLB supérieur ou égal à 8</u>

A titre représentatif et non limitatif des agents tensioactifs non ioniques de HLB supérieur ou égal à 8 à 25 °C pouvant être utilisés seuls ou en mélange dans les compositions de maquillage selon l'invention, on peut citer notamment :

- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination « NEODOL 25-7 »® par SHELL CHEMICALS ;
- les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P par la société ICI UNIQUEMA;
- les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination « Simulsol 220 TM » par la société SEPPIC; le stéarate de glycéryle polyéthoxylé à 30 groupes d'oxyde d'éthylène comme le produit TAGAT S vendu par la société GOLDSCHMIDT, l'oléate de glycéryle polyéthoxylé à 30 groupes d'oxyde d'éthylène comme le produit TAGAT O vendu par la société GOLDSCHMIDT, le cocoate de glycéryle polyéthoxylé à 30 groupes d'oxyde d'éthylène comme le produit VARIONIC LI 13 vendu par la société SHEREX, l'isostéarate de glycéryle polyéthoxylé à 30 groupes d'oxyde d'éthylène comme le produit TAGAT L vendu par la société GOLDSCHMIDT et le laurate de glycéryle polyéthoxylé à 30 groupes d'oxyde d'éthylène comme le produit TAGAT I de la société GOLDSCHMIDT ;
- les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination « Tween 60 » par la société UNIQUEMA ;
- la diméthicone copolyol, telle que celle vendue sous la dénomination « Q2-5220 » par la société DOW CORNING ;
- la diméthicone copolyol benzoate (FINSOLV SLB 101 et 201 de la société FINTEX) ;
- les copolymères d'oxyde propylène et d'oxyde d'éthylène, également appelés polycondensats OE/OP ;
- et leurs mélanges.

Les polycondensats OE/OP sont plus particulièrement des copolymères consistant en des blocs polyéthylène glycol et polypropylène glycol, comme par exemple les polycondensats tribloc polyéthylène glycol / polypropylène glycol / polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

dans laquelle a va de 2 à 120, et b va de 1 à 100.
Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les

polycondensats tribloc polyéthylène glycol / polypropylène glycol / polyéthylène glycol vendus sous les dénominations "SYNPERONIC" comme les "SYNPERONIC PE/ L44" et "SYNPERONIC PE/F127 " par la société ICI, et leurs mélanges.

A cet agent tensioactif non ionique de HLB supérieur ou égal à 8, il peut le cas échéant être associé un ou plusieurs agents tensioactif non ioniques de HLB inférieur à 8 à 25 °C.

A titre représentatif et non limitatif de ces agents ayant un HLB inférieur à 8 à 25 °C, on peut plus particulièrement citer :

- les esters et éthers d'oses tels que les stéarate de sucrose, cocoate de sucrose, stéarate de sorbitan et leurs mélanges comme l'Arlatone 2121 commercialisé par la société ICI ;
- les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M par la société GOLDSCHMIDT, laurate de glycéryle tel que le produit vendu sous la dénomination IMWITOR 312 par la société HULS, stéarate de polyglycéryl-2, tristéarate de sorbitan, ricinoléate de glycéryle ;
- le mélange de cyclométhicone/diméthicone copolyol vendu sous la dénomination "Q2-3225C" par la société DOW CORNING.

La quantité en tensioactif non ionique est généralement ajustée de manière à obtenir une composition possédant les paramètres tels que définis précédemment. La détermination de cette quantité relève des compétences de l'homme de l'art.

A titre illustratif et non limitatif du domaine de l'invention, cette quantité de tensioactif non ionique de HLB supérieur ou égal à 8 peut aller de 0,01 à 40 % en poids, en particulier de 0,1 à 25 en poids, voire de 0,2 à 15 % en poids, et mieux de 0,4 à 10 % en poids par rapport au poids total de la composition.

<u>TENSIOACTIF IONIQUE</u>

Les tensioactifs ioniques mis en oeuvre dans le cadre de la présente invention peuvent être anioniques ou cationiques. Toutefois, généralement, le choix d'au moins un tensioactif anionique est privilégié.

A titre représentatif des tensioactifs anioniques convenant à l'invention, on peut plus particulièrement citer :

- les sels d'acides gras en $C_{16}$-$C_{30}$ notamment ceux dérivant des amines, comme le stéarate de triéthanolamine et/ou le stéarate d'amino-2 méthyl-2 propane di-ol-1,3;
- les sels d'acides gras polyoxyéthylénés notamment ceux dérivant des amines ou les sels alcalins, et leurs mélanges ;
- les esters phosphoriques et leurs sels tels que le "DEA oleth-10 phosphate" (Crodafos N 10N de la société CRODA) ;
- les sulfosuccinates tels que le "Disodium PEG-5 citrate lauryl sulfosuccinate" et le "Disodium ricinoleamido MEA sulfosuccinate"
- les alkyléthersulfates tels que le lauryl éther sulfate de sodium ;
- les iséthionates ;
- les acylglutamates tels que le "Disodium hydrogenated tallow glutamate" (AMISOFT HS-21 R commercialisé par la société AJINOMOTO) et leurs mélanges.

Conviennent tout particulièrement à l'invention, le stéarate de triéthanolamine et/ou le stéarate d'amino-2 méthyl-2 propane di-ol-1,3. Ce dernier est généralement obtenu par simple mélange de l'acide stéarique avec de la triéthanolamine et/ou de l'amino-2 méthyl-2 propane di-ol-1,3.

A titre représentatif des tensioactifs cationiques, on peut notamment citer :

- les alkyl-imidazolidinium tels que l'étho-sulfate d'isostéaryl-éthylimidonium,
- les sels d'ammonium tels que le chlorure de N,N,N-triméthyl-1-docosanaminium (chlorure de Behentrimonium).

Les compositions selon l'invention peuvent également contenir un ou plusieurs tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination « PECOSIL PS 100 » par la société PHOENIX CHEMICAL.

D'une manière générale, les compositions selon l'invention peuvent contenir de 0,01 à 30 % en poids, en particulier de 0,1 à 15 % en poids voire de 0,5 à 10 % en poids de tensioactif ionique par rapport au poids total de la composition.

Cire

La composition selon l'invention peut comprendre une cire ou un mélange de cires.
La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 120 °C.
En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
En particulier, les cires convenant à l'invention peuvent présenter un point de fusion supérieur à 45° environ, mieux supérieur ou égal à 50°C et en particulier supérieur ou égal à 55 °C.
Le point de fusion de la cire peut être mesuré à l'aide d'un calorimètre à balayage différentiel (D.S.C.), par exemple le calorimètre vendu sous la dénomination DSC 30 par la société METLER.
Le protocole de mesure est le suivant :
Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5°C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.
Les cires susceptibles d'être utilisées dans les compositions selon l'invention sont choisies parmi les cires, solides et rigides à température ambiante, d'origine animale, végétale, minérale ou de synthèse et leurs mélanges.
La cire peut également présenter une dureté allant de 0,05 MPa à 30 MPa, et de préférence allant de 6 MPa à 15 MPa . La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.
Le protocole de mesure est le suivant :
La cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté.
La valeur de la dureté est la force de compression maximale mesurée divisée par la surface du cylindre du texturomètre en contact avec la cire.
On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32.
Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.
On peut encore citer les cires de silicone, les cires fluorées.
On peut également utiliser la cire obtenue par hydrogénation d'huile d'olive estérifiée avec l'alcool stéarylique vendue sous la dénomination « PHYTOWAX Olive 18 L 57 » ou bien encore les cires obtenues par hydrogénation d'huile de ricin estérifiée avec l'alcool cétylique vendus sous la dénomination « PHYTOWAX ricin 16L64 et 22L73 », par la société SOPHIM. De telles cires sont décrites dans la demande FR-A- 2792190.
Selon un mode de réalisation particulier, les compositions selon l'invention peuvent comprendre au moins une cire dite cire collante c'est-à-dire possédant un collant supérieur ou égal à 0,7 N.s et une dureté inférieure ou égale à 3,5 MPa.
L'utilisation d'une cire collante peut notamment permettre l'obtention d'une composition cosmétique qui s'applique facilement sur les fibres kératiniques, ayant une bonne accroche sur les fibres kératiniques et qui conduit à la formation d'un maquillage lisse, homogène et épaississant.
La cire collante utilisée peut posséder notamment un collant allant de 0,7 N.s à 30 N.s, en particulier supérieur ou égal

à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination « TA-TX2i® » par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange, en particulier un 12-(12'-hydroxystéaryloxy)stéarate d'alkyle en $C_{20}$-$C_{40}$, de formule (II) :

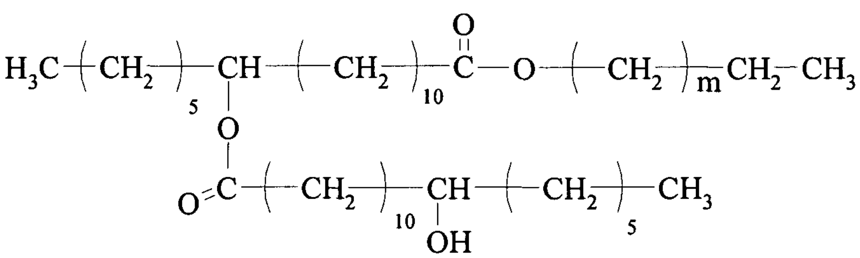

(II)

dans laquelle m est un entier allant de 18 à 38, ou un mélange de composés de formule (II). Une telle cire est notamment vendue sous les dénominations « Kester Wax K 82 P® » et « Kester Wax K 80 P® » par la société KOSTER KEUNEN. Les cires citées ci-dessus présentent généralement un point de fusion commençante inférieur à 45 °C.

La composition selon l'invention peut comprendre une teneur totale en cires allant de 1 à 50% en poids par rapport au poids total de la composition, de préférence de 5 à 40 % en poids, en particulier elle peut en contenir de 10 à 35 %, plus particulièrement de 10 à 30 %.

Dans la présente invention, on peut également utiliser des cires fournies sous forme de petites particules ayant une dimension exprimée en diamètre « effectif » moyen en volume D[4,3] de l'ordre de 0,5 à 30 micromètres, en particulier de 1 à 20 micromètres, et plus particulièrement de 5 à 10 micromètres, désignées par la suite par l'expression « micro cires ».

Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La composition est caractérisée par son diamètre "effectif" moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.
Les mesures sont réalisées à 25 °C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre.
Le diamètre « effectif » est obtenu en prenant un indice de réfraction de 1,33 pour l'eau et un indice de réfraction moyen de 1,42 pour les particules.

Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

Selon un mode de réalisation, la ou les cires (y compris la cire collante) peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 µm.

Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.
Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm). Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Lorsque la cire ou le mélange de cires est présent, dans les compositions selon l'invention, sous la forme d'une dispersion aqueuse de particules, la taille des particules, exprimée en diamètre « effectif » moyen en volume D [4,3] tel que défini ci-après, peut être avantageusement inférieure ou égale à 1 µm et notamment inférieure ou égale à 0,75 µm.
Les particules de cire peuvent présenter des formes variées. Elles peuvent notamment être sphériques.

Selon un mode de réalisation de l'invention, la composition est exempte d'huile de jojoba isomérisée telle que l'huile de jojoba partiellement hydrogénée isomérisée trans fabriquée ou commercialisée par la société Desert Whale sous la référence commerciale ISO-JOJOBA-50®. En effet, on peut supposer, sans être lié par cette explication, que cette cire qui recristallise à température ambiante n'est pas favorable à l'obtention d'un composition d'aspect homogène et lisse.

C'est pourquoi un autre objet de la présente invention est une composition de revêtement des fibres kératiniques comprenant un milieu cosmétiquement acceptable caractérisée en ce qu'elle associe au moins un tensioactif ionique et au moins un tensioactif non ionique de HLB supérieur ou égal à 8 à 25 °C et en ce qu'elle présente une teneur en matière sèche supérieure ou égale à 40% en poids, ladite composition étant exempte d'huile de jojoba isomérisée et ladite composition étant non solide.

Selon un autre mode de réalisation de l'invention, la composition comprend au moins une cire de paraffine, de préférence en une teneur supérieure ou égale à 1%, de préférence supérieure ou égale à 5%, et mieux, supérieure ou égale à 10%.

Ainsi, selon un autre aspect, l'invention a pour objet une composition de revêtement des fibres kératiniques comprenant un milieu cosmétiquement acceptable caractérisée en ce qu'elle associe au moins un tensioactif ionique et au moins un tensioactif non ionique de HLB supérieur ou égal à 8 à 25 °C et en ce qu'elle présente une teneur en matière sèche supérieure ou égale à 40% en poids, ladite composition comprenant au moins une cire de paraffine et ladite composition étant non solide.

La composition selon l'invention peut comprendre un polymère filmogène.

Dans la présente demande, on entend par "polymère filmogène", un polymère apte à former à lui seul ou en présence d'un agent auxiliaire de filmification, un film continu et adhérent sur un support, notamment sur les matières kératiniques comme les cils.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliqueset les monomère styréniques . En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle. Comme monomère styrèniques, on peut citer le styrène, et l'alpha méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique,

l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

La composition peut comprendre un polymère filmogène hydrosoluble choisi parmi :

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique;
- les polymères d'origine naturelle, éventuellement modifiés, tels que :

- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels que l'acide hyaluronique, les chondroïtines sulfate, et leurs mélanges.

Selon un mode de réalisation de la composition selon l'invention, le polymère filmogène peut être un polymère solubilisé dans un milieu organique liquide de la composition comprenant des huiles ou solvants organiques tels que ceux décrits plus loin (on dit alors que le polymère filmogène est un polymère liposoluble).

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle,

l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène peut être également présent dans la composition sous la forme de particules en dispersion dans une phase aqueuse ou dans une phase solvant non aqueuse (milieu organique liquide de la composition), connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » ou « Daitosol 5000 SJ » par la société DAITO KASEY KOGYO; « Syntran 5760 » par la société Interpolymer ou encore les dispersions aqueuses de polyuréthane vendues sous les dénominations « Neorez R-981® » et « Neorez R-974® » par la société AVECIA-NEORESINS, les « Avalure UR-405® », « Avalure UR-410® », « Avalure UR-425® », « Avalure UR-450® », « Sancure 875® », « Sancure 861® », « Sancure 878® » et « Sancure 2060® » par la société GOODRICH, « Impranil 85® » par la société BAYER, «Aquamere H-1511® » par la société HYDROMER; les sulfopolyesters vendus sous le nom de marque « Eastman AQ® » par la société EASTMAN CHEMICAL PRODUCTS, les dispersions vinyliques comme le « Mexomère PAM » et aussi les dispersions acryliques dans l'isododécane comme le « Mexomère PAP » par la société CHIMEX.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

MILIEU COSMETIQUEMENT ACCEPTABLE

Le milieu cosmétiquement acceptable de la composition selon l'invention comprend avantageusement une phase aqueuse, qui peut former la phase continue de la composition.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leur mélanges.

La phase aqueuse (eau et éventuellement le solvant miscible à l'eau) peut être présente, en une teneur allant de 0,1 % à 95 % en poids, par rapport au poids total de la composition, de préférence allant de 1 % à 80 % en poids. Avantageusement, la phase aqueuse est présente en une teneur supérieure ou égale à 20% en poids, de préférence supérieure ou égale à 30% en poids, mieux supérieure ou égale à 40% en poids, et mieux encore supérieure ou égale à 50% en poids par rapport au poids total de la composition.

Les compositions revendiquées peuvent également contenir des ingrédients couramment utilisés dans le domaine du maquillage des fibres kératiniques.

La composition selon l'invention peut notamment comprendre une ou plusieurs huiles.

L'huile peut être choisie parmi les huiles volatiles et/ou les huile non volatiles, et leurs mélanges. La composition comprend avantageusement au moins une huile volatile.

Par " huile volatile", on entend au sens de l'invention une huile susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg).

Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes (8 $10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants volatils fluorés tels que le nonafluorométhoxybutane ou le perfluorométhylcyclopentane.

L'huile volatile peut être présente dans la composition selon l'invention en une teneur allant de 0,1 % à 60 % en poids, de préférence de 0,1 % à 30 % en poids par rapport au poids total de la composition.

La composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R5 + R6 soit ≥ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de

2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;

- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;
Les huiles fluorées utilisables dans l'invention sont notamment des huiles fluorosiliconées, des polyéthers fluorés, des silicones fluorées telles que décrit dans le document EP-A-847752.
Les huiles non volatiles peuvent être présentes dans la composition selon l'invention en une teneur allant de 0,1 à 20 % en poids, de préférence de 0,1 % à 12 % en poids, par rapport au poids total de la composition.

Additifs

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.
Les matières colorantes pulvérulentes peuvent être choisies parmi les pigments et les nacres.
Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.
La composition selon l'invention peut également comprendre une charges choisie parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® (Orgasol de chez Atochem), de poly-β-alanine et de polyéthylène, les poudres de polymères de tétrafluoroéthylène comme le Téflon®, la lauroyl-lysine, l'amidon, le nitrure de bore, les micro sphères creuses polymériques expansées telles que celles de chlorure de polyvinylidène/acrylonitrile comme l'ExpanceL® (Nobel Industrie), les poudres acryliques telles que le polytrap® (Dow Corning), les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (SILICA BEADS® de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
Les charges peuvent représenter de 0,1 à 25 %, et mieux de 1 à 20 % en poids du poids total de la composition.
La composition de l'invention peut comprendre, en outre, tout additif usuellement utilisé en cosmétique tels que les antioxydants, les conservateurs, les parfums, les neutralisants, les gélifiants, les épaississants, les vitamines, les fibres et leurs mélanges.
Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

Par "fibre", il faut comprendre un objet de longueur L et de diamètre D tel que L soit très supérieur à D, D étant le diamètre du cercle dans lequel s'inscrit la section de la fibre. En particulier, le rapport UD (ou facteur de forme) est choisi dans la gamme allant de 3,5 à 2500, de préférence de 5 à 500, et mieux de 5 à 150.
En particulier, les fibres ont une longueur allant de 1 μm à 10 mm, de préférence de 0,1 mm à 5 mm et mieux de 0,3 mm à 3mm .
Les fibres utilisables dans la composition de l'invention peuvent être choisies parmi les fibres rigides ou non rigides, elles peuvent être d'origine synthétique ou naturelle, minérale ou organique.
A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination "KERMEL", " KERMEL TECH" par la société RHODIA ou de poly-(p-phénylène-téréphtalamide) (ou d'aramide) notamment vendues sous la dénomination Kevlar® par la société DUPONT DE NEMOURS.
Les compositions selon l'invention sont généralement obtenues de manière standard par formation à chaud d'une émulsion.
Plus précisément, ces compositions sont obtenues par chauffage de la cire et le ou les tensioactif(s) considéré(s) de HLB ≤ 8 à une température supérieure à la température de fusion de la cire et non supérieure à 100 °C, jusqu'à fusion complète de la cire, puis ajout progressivement de l'eau, et le cas échéant le polymère gélifiant et/ou des tensioactifs de HLB > 8, portée à une température au moins égale à la température précédente, en agitant jusqu'à retour à la température ambiante.
Des ingrédients hydrosolubles peuvent être ajoutés dans l'eau utilisée pour réaliser l'émulsion, ou dans l'émulsion finalement obtenue.
De même, des ingrédients secondaires éventuellement présents dans la composition sont ajoutés selon les cas soit dans les produits de départ, soit dans la composition terminée.
De préférence, la composition selon l'invention est un mascara.
La composition selon l'invention peut être conditionnée dans un ensemble cosmétique comprenant un récipient délimitant au moins un compartiment qui comprend ladite composition, ledit récipient étant fermé par un élément de fermeture.
Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.
L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.
Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).
Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.
Le contenu des brevets ou demandes de brevets cités précédemment sont incorporés par référence dans la présente demande.
Les exemples qui suivent sont présentés à titre illustratif et non limitatif de l'invention.
Les quantités indiquées sont en pourcentage pondéral et exprimées par rapport au poids total de la composition, sauf instructions contraires. Les extrait sec et profil adhésif sont mesurés selon les protocoles décrits plus haut

<u>Exemple 1 : Mascara</u>

amino-2 méthyl-2 propane di-ol-1,3 0,8 %
acide stéarique (c16-18:50/50) 6,6 %

(suite)

cire de Carnauba 3,45 %
cire d'abeille 4,3 %
cire de paraffine 13,8 %
hydroxyéthyl cellulose quaternisée par chlorure de 2,3 époxypropyl triméthyl ammonium 0,1 %
hydroxyéthyl cellulose 0,9 %
polyméthacrylate de sodium dans l'eau à 25 % non stabilisé 1 % (DARVAN 7 de Vanderbilt)
gomme arabique 3,4 %
stéarate de polyéthylène glycol (40 OE) (MYRJ52P d'Uniqema) 0,5 %
triéthanolamine à 99 % 2,4 %
pigments qs %
conservateurs qs
eau déminéralisée stérilisée qsp 100 %

Cette composition présente un extrait sec de 42% et un profil adhésif inférieur à 3.
La formulation ainsi obtenue possède une texture glissante qui s'applique bien sur les cils. Elle possède également un pouvoir adhésif permettant de bonnes propriétés d'épaississement et de séparation des cils.

## Revendications

1. Composition de revêtement des fibres kératiniques comprenant un milieu cosmétiquement acceptable **caractérisée en ce qu'**elle associe au moins un tensioactif ionique et au moins un tensioactif non ionique de HLB supérieur ou égal à 8 à 25 °C et **en ce qu'**elle présente :

   - une teneur en matière sèche supérieure ou égale à 40% en poids et
   - un profil adhésif inférieur ou égal à 3, ledit profil adhésif représentant le rapport du pouvoir adhésif au moment où 20% de ladite composition est évaporé ($PA_{20\%}$) sur le pouvoir adhésif à To (noté $PA_{T0}$, $T_0$ correspondant au moment de l'application de la composition).

2. Composition selon la revendication précédente, **caractérisée en ce que** le profil adhésif va de 0,1 à 3, de préférence de 0,5 à 2,7, mieux de 1 à 2,5, encore mieux de 1,2 à 2,3 et notamment de 1,5 à 2.

3. Composition de revêtement des fibres kératiniques comprenant un milieu cosmétiquement acceptable **caractérisée en ce qu'**elle associe au moins un tensioactif ionique et au moins un tensioactif non ionique de HLB supérieur ou égal à 8 à 25 °C et **en ce qu'**elle présente une teneur en matière sèche supérieure ou égale à 40% en poids, ladite composition comprenant au moins une cire de paraffine et ladite composition étant non solide.

4. Composition de revêtement des fibres kératiniques comprenant un milieu cosmétiquement acceptable **caractérisée en ce qu'**elle associe au moins un tensioactif ionique et au moins un tensioactif non ionique de HLB supérieur ou égal à 8 à 25 °C et **en ce qu'**elle présente une teneur en matière sèche supérieure ou égale à 40% en poids, ladite composition comprenant au moins une cire de paraffine en une teneur supérieure ou égale à 1 % et ladite composition étant non solide.

5. Composition selon la revendication précédente, **caractérisée en ce que** la cire de paraffine est présente en une quantité supérieure ou égale à 5%, de préférence, supérieure ou égale à 10%.

6. Composition de revêtement des fibres kératiniques comprenant un milieu cosmétiquement acceptable **caractérisée en ce qu'**elle associe au moins un tensioactif ionique et au moins un tensioactif non ionique de HLB supérieur ou égal à 8 à 25 °C et **en ce qu'**elle présente une teneur en matière sèche supérieure ou égale à 40% en poids, ladite composition comprenant étant exempte d'huile de jojoba isomérisée et ladite composition étant non solide.

7. Composition selon l'une quelconque des revendications précédentes, **caractérisée en ce qu'**elle comprend une cire ou un mélange de cires.

8. Composition selon la revendication 7, **caractérisée en ce que** la cire est choisie parmi les cires d'origine animale

ou végétale et leurs mélanges.

**9.** Composition selon la revendication 7 ou 8, **caractérisée en ce que** la cire est choisie parmi les cires hydrocarbonées comme la cire d'abeilles, la cire de lanoline, et les cires d'insectes de Chine ; la cire de riz, la cire de Carnauba, la cire de Candellila, la cire d'Ouricurry, la cire d'Alfa, la cire de fibres de liège, la cire de canne à sucre, la cire du Japon et la cire de sumac ; la cire de montan ; les cires microcristallines, les paraffines et l'ozokérite ; les cires de polyéthylène ; les cires obtenues par la synthèse de Fisher-Tropsch ; les copolymères cireux ainsi que leurs esters ; les cires obtenues par hydrogénation catalytique d'huiles animales ou végétales ayant des chaînes grasses, linéaires ou ramifiées, en C8-C32 comme l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) et le tétrabéhénate de di-(triméthylol-1,1,1 propane) et leurs mélanges.

**10.** Composition selon l'une des revendications 7 à 9, **caractérisée en ce que** la cire est présente à raison de 1 à 50%, de préférence de 5 à 40 %, mieux de 10 à 35 % et notamment de 10 à 30 % en poids du poids total de la composition.

**11.** Composition selon l'une des revendications précédentes, **caractérisée en ce que** le tensioactif non ionique de HLB supérieur ou égal à 8 est choisi parmi les éthers d'alcools gras oxyéthylénés et/ou oxypropylénés, les esters d'acide gras et de polyéthylène glycol, les éthers de glycérol oxyéthylénés et/ou oxypropylénés, les esters d'acide gras et d'éther de sorbitol oxyéthylénés et/ou oxypropylénés et les copolymères d'oxyde propylène et d'oxyde d'éthylène et leurs mélanges.

**12.** Composition selon la revendication 11, **caractérisée en ce qu'**il est choisi parmi les éthers oxyéthylénés de l'alcool cétéarylique à 30 groupes oxyéthylénés, éther oxyéthyléné d'un mélange d'alcools gras en $C_{12}$-$C_{15}$ comportant 7 groupes oxyéthylénés, stéarate de PEG-50, stéarate de PEG-40, stéarate de PEG-200 glycéryle, polysorbate 60, stéarate de glycéryle polyéthoxylé à 30 groupes d'oxyde d'éthylène, oléate de glycéryle polyéthoxylé à 30 groupes d'oxyde d'éthylène, cocoate de glycéryle polyéthoxylé à 30 groupes d'oxyde d'éthylène, isostéarate de glycéryle polyéthoxylé à 30 groupes d'oxyde d'éthylène, laurate de glycéryle polyéthoxylé à 30 groupes d'oxyde d'éthylène, diméthicone copolyol, diméthicone copolyol benzoate et leurs mélanges.

**13.** Composition selon l'une quelconque des revendications précédentes **caractérisée en ce que** le tensioactif non ionique de HLB supérieur ou égal à 8 est présent à raison de 0,01 % à 40 % en poids, en particulier de 0,1 % à 25 % en poids du poids total de la composition.

**14.** Composition selon l'une quelconque des revendications précédentes, **caractérisée en ce que** le tensioactif ionique est au moins un tensioactif anionique.

**15.** Composition selon la revendication 14, **caractérisée en ce que** ledit tensioactif anionique est choisi parmi les sels d'acides gras en $C_{16}$-$C_{30}$ ; les sels d'acides gras polyoxyéthoxylénés ; les esters phosphoriques et leurs sels ; les alkyléthersulfates ; les sulfosuccinates ; les iséthionates et les acylglutamates et leurs mélanges.

**16.** Composition selon l'une quelconque des revendications précédentes **caractérisée en ce que** le tensioactif ionique comprend au moins le stéarate de triéthanolamine et/ou le stéarate d'amino-2 méthyl-2 propane di-ol-1,3.

**17.** Composition selon l'une quelconque des revendications précédentes, **caractérisée en ce que** le tensioactif ionique est présent à raison de 0,01 à 30 % en poids et notamment de 0,1 à 15 % en poids du poids total de la composition.

**18.** Composition selon l'une des revendications précédentes, **caractérisée en ce qu'**elle comprend de 0,1 à 40 % et notamment de 0,3 à 20 % en poids de tensioactifs.

**19.** Composition selon l'une quelconque des revendications précédentes, **caractérisée en ce qu'**elle comprend un polymère filmogène.

**20.** Composition selon la revendication 17, **caractérisée en ce que** le polymère filmogène est choisi parmi les polymères synthétiques, de type radicalaire ou de type polycondensat, les polymères d'origine naturelle et leurs mélanges.

**21.** Composition selon la revendication ou 19 ou 20, **caractérisée en ce que** le polymère filmogène est présent en une teneur en matières sèches allant de 0,1 % à 60 % en poids par rapport au poids total de la composition, de préférence de 0,5 % à 40 % en poids, et mieux de 1 % à 30 % en poids.

**22.** Composition selon l'une des revendications précédentes, **caractérisée en ce qu'**elle comprend une phase aqueuse.

**23.** Composition selon la revendication 22, **caractérisée en ce que** la phase aqueuse est présente en une teneur allant de 0,1 % à 95 % en poids, par rapport au poids total de la composition, de préférence allant de 1 % à 80 % en poids.

**24.** Composition selon l'une des revendications précédentes, **caractérisée en ce qu'**elle comprend une matière colorante.

**25.** Composition selon la revendication 24, **caractérisée en ce que** la matière colorante est présente en une teneur allant de 0,01 % à 30 % en poids, par rapport au poids total de la composition.

**26.** Composition selon l'une des revendications précédentes, **caractérisée en ce qu'**elle a une teneur en matière sèche supérieure ou égale à 41 % en poids, de préférence supérieure ou égale à 42% en poids par rapport au poids total de la composition.

**27.** Composition selon l'une des revendications 1 à 26, **caractérisée en ce qu'**elle est un mascara.

**28.** Utilisation d'une composition selon l'une des revendications 1 à 27, pour obtenir un maquillage chargeant des fibres kératiniques et notamment des cils.

**29.** Utilisation de l'association d'au moins un tensioactif ionique et au moins un tensioactif non ionique de HLB supérieur ou égal à 8 à 25 °C pour obtenir une composition de revêtement des fibres kératiniques ayant une teneur en matière sèche supérieure ou égale à 40% en poids et un profil adhésif inférieur ou égal à 3, ladite composition permettant un maquillage chargeant des fibres kératiniques.

**30.** Procédé cosmétique de traitement ou de maquillage des fibres kératiniques comprenant l'application sur les dites fibres kératiniques d'une composition selon l'une quelconque des revendications 1 à 29.

Office européen des brevets

# RAPPORT DE RECHERCHE EUROPEENNE

Numéro de la demande
EP 05 29 0182

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 833 163 A (L'OREAL) 13 juin 2003 (2003-06-13) * le document en entier * ----- | 1-30 | A61K7/032 |
| | | | **DOMAINES TECHNIQUES RECHERCHES (Int.Cl.7)** |
| | | | A61K |

Le présent rapport a été établi pour toutes les revendications

| Lieu de la recherche | Date d'achèvement de la recherche | Examinateur |
|---|---|---|
| Berlin | 26 mai 2005 | Alvarez Alvarez, C |

1

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 0182

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-05-2005

| Document brevet cité au rapport de recherche | Date de publication | Membre(s) de la famille de brevet(s) | Date de publication |
|---|---|---|---|
| FR 2833163 A | 13-06-2003 | FR 2833163 A1 | 13-06-2003 |

EPO FORM P0460

Pour tout renseignement concernant cette annexe : voir Journal Officiel de l'Office européen des brevets, No.12/82